(19) Europäisches Patentamt
European Patent Office
Office européen des brevets

(11) EP 0 862 946 A1

(12) EUROPÄISCHE PATENTANMELDUNG

(43) Veröffentlichungstag:
**09.09.1998 Patentblatt 1998/37**

(21) Anmeldenummer: **98103948.0**

(22) Anmeldetag: **05.03.1998**

(51) Int. Cl.[6]: **B01J 21/06**, B01J 23/72, B01J 23/755, C07C 45/29, C07C 29/14, C07C 29/143

(84) Benannte Vertragsstaaten:
**AT BE CH DE DK ES FI FR GB GR IE IT LI LU MC NL PT**
Benannte Erstreckungsstaaten:
**AL LT LV MK RO SI**

(30) Priorität: **07.03.1997 CH 540/97**
**06.05.1997 CH 1059/97**

(71) Anmelder: **LONZA AG**
**CH-3945 Gampel/Wallis (CH)**

(72) Erfinder:
- **Heveling, Josef, Dr.**
**3904 Naters (Kanton Wallis) (CH)**
- **Laffan, David, Dr.**
**Macclesfield, Cheshire SK105LW (GB)**
- **Wellig, Alain**
**3986 Ried-Mörel (Kanton Wallis) (CH)**

(74) Vertreter:
**Winter, Brandl & Partner**
**Patent- und Rechtsanwaltskanzlei**
**Alois-Steinecker-Strasse 22**
**85354 Freising (DE)**

# (54) Wasserstofftransferkatalysator auf der Basis von Kupfer und/oder Nickel dotiertem Zirkoniumhydroxid

(57) Beschrieben werden Katalysatorzusammensetzungen auf Basis von amorphem teildehydratisiertem Zirconiumhydroxid, die mit 0,01 bis 20 Atom-% Kupfer und/oder 0,01 bis 20 Atom-% Nickel, jeweils bezogen auf Zirconium, dotiert sind und eine spezifische Oberfläche nach BET von mindestens 50 $m^2/g$ besitzen. Die erfindungsgemässen Katalysatorzusammensetzungen eignen sich insbesondere als Katalysator in Wasserstofftransferreaktionen wie der Meerwein—Ponndorf—Verley-Reduktion oder der Oppenauer-Oxidation. Weiterhin wird die Herstellung von 3-Hydroxychinuclidin der Formel

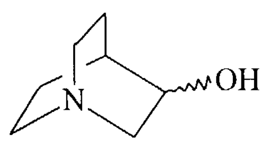

durch Umsetzung von Chinuclidin-3-on mit einem sekundären Alkohol in Gegenwart von amorphem teildehydratisiertem Zirconiumhydroxid beschrieben.

EP 0 862 946 A1

## Beschreibung

Die vorliegende Erfindung betrifft Katalysatoren auf der Basis von amorphem teildehydratisiertem Zirconiumhydroxid ($ZrO_2 \cdot x\ H_2O$), ein Verfahren zu deren Herstellung, sowie deren Verwendung in Wasserstofftransferreaktionen zwischen Carbonylverbindungen und Alkoholen, insbesondere der Meerwein—Ponndorf—Verley-Reduktion und der Oppenauer-Oxidation. Sie betrifft weiterhin ein Verfahren zur Herstellung von 3-Hydroxychinuclidin der Formel

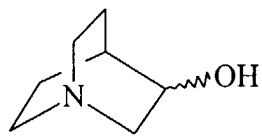

I

durch Reduktion von Chinuclidin-3-on der Formel

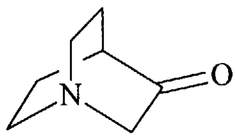

II.

Es ist bekannt, dass die üblicherweise unter der katalytischen Wirkung von Aluminiumalkoxiden durchgeführten und unter den Namen „Meerwein—Ponndorf—Verley-Reduktion" und „Oppenauer-Oxidation" zusammengefassten Wasserstofftransferreaktionen zwischen Aldehyden oder Ketonen und primären oder sekundären Alkoholen auch unter heterogener Katalyse durchgeführt werden können (C. F. de Graauw et al., *Synthesis* **1994**, 1007—1017). Wegen der leichteren Aufarbeitung des Reaktionsgemisches und der unter Umständen möglichen Wiederverwendung des Katalysators ist diese Variante von grossem Interesse. Als heterogener Katalysator wurde hierbei unter anderem auch teildehydratisiertes Zirconiumhydroxid („hydrous zirconium oxide") eingesetzt (M. Shibagaki et al., *Bull. Chem. Soc. Jpn.* **1988**, *61*, 3283—3288; *Bull. Chem. Soc. Jpn.* **1988**, *61*, 4153—4154; *Bull. Chem. Soc. Jpn.* **1990**, *63*, 258—259; H. Kuno et al., *Bull. Chem. Soc. Jpn.* **1990**, *63*, 1943—1946; *Bull. Chem. Soc. Jpn.* **1991**, *63*, 312—314). Die Aktivität dieses Katalysators ist jedoch nicht sehr hoch, so dass insbesondere mit wenig reaktiven Carbonylverbindungen und/oder Alkoholen häufig nur mässige oder schlechte Ausbeuten erhalten werden.

3-Hydroxychinuclidin ist ein Ausgangsmaterial für die Synthese verschiedener pharmazeutischer Wirkstoffe wie beispielsweise Cholinomimetika (US-A-2 648 667, EP-A-0 370 415) oder Bronchodilatoren (WO-A-93/06 098). Zur Herstellung von 3-Hydroxychinuclidin aus Chinuclidin-3-on sind verschiedene Verfahren bekannt (US-A-2 648 667), nämlich die Hydrierung des Hydrochlorids mit Platinoxid als Katalysator, die Reduktion des Hydrochlorids mit Natrium/Ethanol, die Hydrierung der freien Base mit Platinoxid oder Raney-Nickel als Katalysator, sowie die Reduktion der freien Base mit Lithiumaluminiumhydrid. Keines dieser Verfahren ist jedoch frei von Nachteilen. Platinoxid ist sehr teuer, Raney-Nickel ist pyrophor und Natrium/Ethanol sowie Lithiumaluminiumhydrid als Reduktionsmittel führen bei Anwendung im technischen Massstab zu Sicherheits- und Abfallproblemen.

Aufgabe der vorliegenden Erfindung war daher zum einen, aktivere heterogene Katalysatoren für die Meerwein—Ponndorf—Verley-Reduktion und die Oppenauer-Oxidation bereitzustellen. Zum anderen war Aufgabe der vorliegenden Erfindung, ein alternatives Verfahren zur Herstellung von 3-Hydroxychinuclidin bereitzustellen, welches sich zur Durchführung in technischem Massstab eignet, wenig Abfall liefert und weder hohen Druck noch teure oder gefährliche Reagenzien erfordert.

Erfindungsgemäss wird diese Aufgabe durch die Katalysatoren nach Patentanspruch 1 und das Verfahren zur Herstellung von 3-Hydroxychinuclidin nach Patentanspruch 13 gelöst.

Es wurde gefunden, dass die Aktivität von amorphem teildehydratisiertem Zirconiumhydroxid mit einer spezifischen Oberfläche nach BET von mindestens 50 $m^2/g$ durch Dotierung mit 0,01 bis 20 Atom% (bezogen auf Zr) Kupfer und/oder Nickel erheblich gesteigert werden kann.

Vorzugsweise entsprechen die erfindungsgemässen Katalysatorzusammensetzungen der Formel

$$Cu_aNi_bZrO_{2+a+b} \cdot x\ H_2O.$$

Hierin ist $0 \leq a \leq 0{,}2$; $0 \leq b \leq 0{,}2$ und $0{,}2 \leq x \leq 2{,}0$, mit der Massgabe, dass $a + b \geq 0{,}0001$ ist.

Besonders bevorzugt sind solche Katalysatorzusammensetzungen, in denen $a \leq 0{,}15$; $b \leq 0{,}1$; $a + b \geq 0{,}0002$ und $0{,}3 \leq x \leq 2{,}0$ ist.

Insbesondere sind solche Katalysatorzusammensetzungen bevorzugt, in welchen entweder Kupfer oder Nickel enthalten ist, worin also entweder $a = 0$ oder $b = 0$ ist.

Die spezifische Oberfläche nach BET der erfindungsgemässen Katalysatorzusammensetzungen ist vorzugsweise grösser als 100 $m^2/g$; besonders bevorzugt sind Katalysatorzusammensetzungen mit einer spezifischen Oberfläche von mehr als 150 $m^2/g$.

Die erfindungsgemässen Katalysatorzusammensetzungen können beispielsweise dadurch hergestellt werden, dass aus einer wässrigen Lösung eines Zirconiumsalzes durch Zugabe einer Base Zirconiumhydroxid ausgefallt, bei 200 bis 400 °C kalziniert und anschliessend mit einer Lösung eines Kupfer- und/oder Nickelsalzes imprägniert und getrocknet wird.

Vorzugsweise wird als Zirconiumsalz Zirconylchlorid ($ZrOCl_2$) und als Base wässrige Ammoniaklösung oder eine wässrige Alkalihydroxidlösung wie beispielsweise Natronlauge eingesetzt.

Als Kupfer und/oder Nickelsalz wird vorzugsweise das entsprechende Nitrat eingesetzt.

Die Kalzinationstemperatur liegt vorzugsweise bei 250 bis 350 °C, besonders bevorzugt bei 270 bis 320 °C.

Die erfindungsgemässen Katalysatorzusammensetzungen eignen sich als Katalysator für die Reduktion von Aldehyden oder Ketonen zu den entsprechenden primären bzw. sekundären Alkoholen durch Wasserstoffübertragung von einem sekundären Alkohol als Wasserstoffdonor (Meerwein—Ponndorf—Verley-Reduktion). Als Wasserstoffdonor wird hierbei vorzugsweise Isopropylalkohol eingesetzt.

Die erfindungsgemässen Katalysatorzusammensetzungen eignen sich ebenfalls als Katalysator für die Oxidation von primären oder sekundären Alkoholen zu den entsprechenden Aldehyden oder Ketonen durch Wasserstoffübertragung auf ein Keton oder Chinon als Wasserstoffakzeptor (Oppenauer-Oxidation).

Als Wasserstoffakzeptor wird vorzugsweise Cyclohexanon oder *p*-Benzochinon eingesetzt.

Die erfindungsgemässen Katalysatorzusammensetzungen lassen sich ohne weiteres mehrmals verwenden, ohne dass eine nennenswerter Verlust an Aktivität eintritt oder ein signifikanter Teil der Dotierung verlorengeht.

Es wurde weiterhin gefunden, dass Chinuclidin-3-on oder ein entsprechendes Salz wie beispielsweise das Chinuclidin-3-on-Hydrochlorid mit einem sekundären Alkohol als Wasserstoffdonor in Gegenwart von amorphem teildehydratisiertem Zirconiumhydroxid ($ZrO_2 \cdot x\ H_2O$) nach Art einer Meerwein—Ponndorf—Verley-Reduktion in guter Ausbeute zu 3-Hydroxychinuclidin bzw. dem entsprechenden Salz reduziert werden kann. Falls die Reduktion mit einem Salz des Chinuclidin-3-ons durchgeführt wird, kann das so erhältliche Salz des 3-Hydroxychinuclidins gegebenenfalls bei der Aufarbeitung des Reaktionsgemisches durch Zugabe einer starken Base in das freie 3-Hydroxychinuclidin übergeführt werden.

Das amorphe teildehydratisierte Zirconiumhydroxid kann beispielsweise durch Fällung von Zirconiumhydroxid aus einer wässrigen Zirconiumsalzlösung und anschliessende Kalzination bei niedriger Temperatur hergestellt werden. Als Zirconiumsalzlösung eignet sich beispielsweise eine Zirconylchloridlösung, als Fällungsmittel beispielsweise eine Alkalilauge. Die Kalzination kann beispielsweise bei 270—320 °C durchgeführt werden.

Als sekundärer Alkohol wird im erfindungsgemässen Verfahren vorzugsweise Isopropylalkohol eingesetzt. Dieser wird dabei zu Aceton dehydriert. Da es sich bei der Reaktion um eine Gleichgewichtsreaktion handelt, wird der sekundäre Alkohol vorteilhaft im Überschuss eingesetzt, um das Gleichgewicht in die gewünschte Richtung zu verschieben. Der überschüssige sekundäre Alkohol kann dabei gleichzeitig als Lösungsmittel dienen.

Die Reaktion wird vorzugsweise bei einer Temperatur von 120—220 °C, besonders bevorzugt bei 150—200 °C in der Flüssigphase durchgeführt. Besitzt der eingesetzte sekundäre Alkohol bei Normaldruck einen Siedepunkt unterhalb der Reaktionstemperatur, dann wird die Reaktion zweckmässig bei erhöhtem Druck in einem Autoklaven oder einem anderen geeigneten Druckbehälter durchgeführt.

Der Katalysator kann nach dem Ende der Reaktion durch Filtration sehr einfach abgetrennt und (gegebenenfalls nach einem Waschvorgang) ohne grossen Aktivitätsverlust wiederverwendet werden.

Die nachfolgenden Beispiele verdeutlichen die Erfindung, ohne dass darin eine Einschränkung zu sehen ist.

## Beispiel 1

### Herstellung von $ZrO_2 \cdot x\ H_2O$

In einem mit einem Hochgeschwindigkeitsrührer (Ultra-Turrax®, 9000 $min^{-1}$) ausgerüsteten Durchflussreaktor wurde Zirconylchloridlösung (265 g/l, gerechnet als $ZrO_2$) mit Natronlauge (30%) halbkontinuierlich ausgefällt. Die Zugabegeschwindigkeit der Zirconylchloridlösung wurde so eingestellt, dass innerhalb von 5 h 50 kg $ZrO_2$ eingebracht wurden. Die Natronlauge wurde über eine Dosierpumpe so schnell zugegeben, dass die Fällung bei einem konstanten pH von 8,0 stattfand. Da hierfür weniger als die stöchiometrische Menge NaOH benötigt wurde, gab man den Rest der

stöchiometrischen Menge nach der Fällung in die erhaltene Zirconiumhydroxidsuspension. In einer Kammerfilterpresse wurde die Suspension anschliessend entwässert und der Filterkuchen mit entsalztem Wasser neutral und chloridfrei gewaschen. Der gewaschene Filterkuchen wurde bei 100 °C getrocknet und dann in Wasser eingetragen, wobei die Filterkuchenstücke in kleine Teile zersprangen. Diese wurden nochmals bei 100 °C getrocknet und anschliesssend mit 30 K/h auf 300 °C erhitzt und bei dieser Temperatur 8 h kalziniert. Das so erhaltene Produkt war röntgenamorph, hatte eine spezifische Oberfläche nach BET von 196 $m^2/g$ und ein Porenvolumen von 0,43 $cm^3/g$.

**Beispiel 2**

**Herstellung von $ZrO_2 \cdot x\ H_2O$**

Es wurde verfahren wie in Beispiel 1 beschrieben, jedoch betrug die Kalzinationstemperatur nur 270 °C. Das so erhaltene Produkt war röntgenamorph, hatte eine spezifische Oberfläche nach BET von 212 $m^2/g$ und ein Porenvolumen von 0,41 $cm^3/g$. Der durch Thermogravimetrie bestimmte Wassergehalt entsprach der Formel $ZrO_2 \cdot 0{,}57\ H_2O$. Bei 430 °C wandelte es sich exotherm in eine kristalline Form um.

**Beispiel 3**

**Herstellung von $ZrO_2 \cdot x\ H_2O$**

Zirconylchlorid-Octahydrat ($ZrOCl_2 \cdot 8\ H_2O$) wurde in Wasser gelöst. Die leichte Trübung wurde abfiltriert und das Filtrat mit entsalztem Wasser auf einen Gehalt von 50 g/l (gerechnet als $ZrO_2$) eingestellt. In einem Reaktionsgefäss mit Hochgeschwindigkeitsrührer wurden 2,5 l entsalztes Wasser vorgelegt. Unter heftigem Rühren (8000 $min^{-1}$) wurden 50 ml/min der Zirkonylchloridlösung und soviel 10%ige Ammoniaklösung simultan zudosiert, dass während der resultierenden Fällung ein pH von 7,0 ± 0,2 eingehalten wurde. Gleichzeitig wurde soviel entsalztes Wasser zugegeben, dass der Feststoffgehalt der Suspension ca. 1% nicht überstieg. Nach beendeter Fällung wurde der Feststoff durch Filtration abgetrennt und der Filterkuchen solange mit Ammoniakwasser gewaschen, bis der Chloridgehalt auf 0,05% gesunken war. Der gewaschene Filterkuchen wurde bei 100 °C getrocknet, nochmals in Wasser aufgeschlämmt und erneut filtriert und getrocknet. Das so erhaltene Zirconiumoxidhydrat wurde während 8 h bei 300 kalziniert. Das so erhaltene Produkt war röntgenamorph und hatte eine spezifische Oberfläche nach BET von 240 $m^2/g$.

**Beispiel 4**

**$Cu_{0,03}ZrO_{2,03} \cdot 0{,}61\ H_2O$**

20 g $ZrO_2 \cdot x\ H_2O$ aus Beispiel 3 wurden mit je 40 ml einer 1,027 M Kupfer(II)nitratlösung 2 × 16 h behandelt und nach jeder Behandlung zehnmal mit Wasser gewaschen und 12 h bei 120 °C/30 mbar getrocknet. Die so erhaltene Katalysatorzusammensetzung hatte einen Kupfergehalt von 1,4 Gew.% und einen Glühverlust von 8,1%, entsprechend der Formel $Cu_{0,03}ZrO_{2,03} \cdot 0{,}61\ H_2O$. Die spezifische Oberfläche nach BET betrug 247 $m^2/g$, das Porenvolumen 0,24 $cm^3/g$. Die Kristallisation setzte bei 497 °C ein.

**Beispiel 5**

**$Ni_{0,013}ZrO_{2,013} \cdot 0{,}62\ H_2O$**

Es wurde verfahren wie in Beispiel 4 beschrieben, jedoch wurde anstelle der Kupfer(II)nitratlösung 200 ml 0,5 M Nickel(II)nitratlösung eingesetzt und die Dauer der ersten Behandlung auf 2 h verkürzt. Nach der ersten Behandlung wurde 15mal mit Wasser gewaschen, nach der zweiten Behandlung siebenmal. Die so erhaltene Katalysatorzusammensetzung hatte einen Nickelgehalt von 0,6 Gew.% und einen Glühverlust von 8,2%, entsprechend der Formel $Ni_{0,013}ZrO_{2,013} \cdot 0{,}62\ H_2O$. Die spezifische Oberfläche nach BET betrug 248 $m^2/g$, das Porenvolumen 0,23 $cm^3/g$. Die Kristallisation setzte bei 495 °C ein.

**Beispiel 6**

**$Cu_{0,0047}ZrO_{2,0047} \cdot 0{,}39\ H_2O$**

In einem 1000 ml-Kolben wurden 100 g $ZrO_2 \cdot x\ H_2O$ (hergestellt nach Beispiel 1) mit 400 ml einer 0,276 M Kupfer(II)nitratlösung 72 h unter langsamer Rotation (Rotationsverdampfer) vermischt. Anschliessend wurde die so erhaltene Katalysatorsuspension über eine Nutsche filtriert und der Filterkuchen 25 mal mit entsalztem Wasser aufgeschlämmt und jeweils erneut filtriert. Anschliessend wurde der Filterkuchen noch 72 h im Soxhlet-Extraktor mit entsalztem Wasser extrahiert und schliesslich 24 h bei 120 °C/30 mbar getrocknet. Die so erhaltene Katalysatorzusammensetzung hatte einen Kupfergehalt von 0,23 Gew.% und einen Glühverlust von 5,4%, entsprechend der Formel $Cu_{0,0047}ZrO_{2,0047} \cdot 0,39\ H_2O$. Die spezifische Oberfläche nach BET betrug 219 $m^2/g$, das Porenvolumen 0,44 $cm^3/g$. Die Kristallisation setzte bei 408 °C ein.

**Beispiel 7**

**$Ni_{0,00022}ZrO_{2,00022} \cdot 0,35\ H_2O$**

Es wurde verfahren wie in Beispiel 6 beschrieben, jedoch wurde anstelle der Kupfer(II)nitratlösung eine 0,256 M Nickel(II)nitratlösung eingesetzt. Die so erhaltene Katalysatorzusammensetzung hatte einen Nickelgehalt von 0,01 Gew.% und einen Glühverlust von 4,9%, entsprechend der Formel $Ni_{0,00022}ZrO_{2,00022} \cdot 0,35\ H_2O$. Die spezifische Oberfläche nach BET betrug 217 $m^2/g$, das Porenvolumen 0,44 $cm^3/g$. Die Kristallisation setzte bei 404 °C ein.

**Beispiel 8**

**$Cu_{0,016}ZrO_{2,016} \cdot 0,50\ H_2O$**

50 g $ZrO_2 \cdot x\ H_2O$ aus Beispiel 2 wurden mit je 200 ml einer 0,505 M Kupfer(II)nitratlösung 2 × 24 h behandelt, nach jeder Behandlung fünfmal mit Wasser gewaschen und schliesslich 24 h bei 120 °C/30 mbar getrocknet. Die so erhaltene Katalysatorzusammensetzung hatte einen Kupfergehalt von 0,74 Gew.% und einen Glühverlust von 6,8%, entsprechend der Formel $Cu_{0,016}ZrO_{2,016} \cdot 0,50\ H_2O$. Die spezifische Oberfläche nach BET betrug 214 $m^2/g$, das Porenvolumen 0,41 $cm^3/g$. Die Kristallisation setzte bei 456 °C ein.

**Beispiel 9**

**$Cu_{0,053}ZrO_{2,053} \cdot 1,35\ H_2O$**

100 g $ZrO_2 \cdot x\ H_2O$ (Typ XZO 631/02 der Firma MEL Chemicals, Manchester, UK; spezifische Oberfläche nach BET 210 $m^2/g$, Porenvolumen 0,12 $cm^3/g$, Kristallisation ab 419 °C) wurden mit 200 ml einer 0,513 M Kupfer(II)nitratlösung 24 h behandelt, danach sechsmal mit Wasser gewaschen und schliesslich 24 h bei 100 °C/30 mbar getrocknet. Die so erhaltene Katalysatorzusammensetzung hatte einen Kupfergehalt von 2,17 Gew.% und einen Glühverlust von 16,0%, entsprechend der Formel $Cu_{0,053}ZrO_{2,053} \cdot 1,35\ H_2O$. Die spezifische Oberfläche nach BET betrug 274 $m^2/g$, das Porenvolumen 0,12 $cm^3/g$. Die Kristallisation setzte bei 508 °C ein.

**Beispiel 10**

**$Cu_{0,067}ZrO_{2,067} \cdot 1,40\ H_2O$**

Es wurde verfahren wie in Beispiel 9 beschrieben, jedoch wurden 250 g $ZrO_2 \cdot x\ H_2O$ und 500 ml 0,513 M Kupfer(II)nitratlösung eingesetzt. Die Zahl der Waschvorgänge wurde auf fünf reduziert. Die so erhaltene Katalysatorzusammensetzung hatte einen Kupfergehalt von 2,75 Gew.%. Der Glühverlust von 16,4% entsprach der Formel $Cu_{0,067}ZrO_{2,067} \cdot 1,40\ H_2O$. Die spezifische Oberfläche nach BET betrug 229 $m^2/g$, das Porenvolumen 0,10 $cm^3/g$. Die Kristallisation setzte bei 520 °C ein.

**Beispiel 11**

**$Cu_{0,14}ZrO_{2,14} \cdot 1,90\ H_2O$**

Es wurde verfahren wie in Beispiel 9 beschrieben, jedoch wurden 50 g $ZrO_2 \cdot x\ H_2O$ und 120 ml 0,529 M Kupfer(II)nitratlösung eingesetzt. Das Produkt wurde nicht gewaschen. Die so erhaltene Katalysatorzusammensetzung hatte einen Kupfergehalt von 5,30 Gew.% und einen Glühverlust von 20,3%, entsprechend der Formel $Cu_{0,14}ZrO_{2,14} \cdot 1,90\ H_2O$. Die spezifische Oberfläche nach BET betrug 229 $m^2/g$, das Porenvolumen 0,10 $cm^3/g$. Die Kristallisation setzte bei 564 °C ein.

**Beispiele 12—13, Vergleichsbeispiel 1**

***trans*-2-Hexenol (Meerwein—Ponndorf—Verley-Reduktion)**

In einem Rundkolben mit Magnetrührer und Rückflusskühler wurden 0,5 g (5,1 mmol) *trans*-2-Hexenal, 12 g Isopropylalkohol und 3,9 g Katalysator zum Rückfluss erhitzt. Nach dem Ende der Reaktionsdauer wurde das Reaktionsgemisch gaschromatographisch analysiert und die Ausbeute bestimmt. Die Ergebnisse sind in der folgenden Tabelle 1 zusammengefasst.

Tabelle 1

| Beispiel Nr. | Katalysator | Reaktionsdauer [h] | Ausbeute [%] |
|---|---|---|---|
| V 1 | aus Beispiel 3 (undotiert) | 8 | 77,4 |
| 12 | aus Beispiel 4 | 7 | 88,0 |
| 13 | aus Beispiel 5 | 7 | 79,2 |

**Beispiele 14—20, Vergleichsbeispiele 2—3**

**Zimtalkohol (Meerwein—Ponndorf—Verley-Reduktion)**

In einem Dreihalskolben mit Rückflusskühler und Magnetrührer wurden unter Argon 0,5 g (3,8 mmol) Zimtaldehyd (*trans*-3-Phenyl-2-propenal) und 12 g Isopropylalkohol (für ein Verhältnis Edukt/Katalysator = 1:1) bzw. 1,5 g (11,3 mmol) Zimtaldehyd und 36 g Isopropylalkohol (Edukt/Katalysator = 3:1) mit 0,5 g Katalysator und 1,5 g Dodecan (als GC-Standard) zum Rückfluss erhitzt. Nach dem Ende der Reaktionsdauer wurde das Reaktionsgemisch gaschromatographisch analysiert und die Ausbeute bestimmt. Die Ergebnisse sind in der folgenden Tabelle 2 zusammengefasst.

Tabelle 2

| Beispiel Nr. | Katalysator | Reaktionsdauer [h] | Ausbeute [%] | Edukt/Kat. |
|---|---|---|---|---|
| V 2 | aus Beispiel 1 (undotiert) | 24 | 63,8 | 1:1 |
| V 3 | MEL*) (undotiert) | 24 | 44,5 | 3:1 |
| 14 | aus Beispiel 6 | 24 | 72,4 | 1:1 |
| 15 | aus Beispiel 4 | 24 | 97,3 | 1:1 |
| 16 | aus Beispiel 9 | 22,3 | 100,0 | 3:1 |
| 17 | aus Beispiel 9 | 19,2 | 98,5 | 3:1 |
| 18 | aus Beispiel 10 | 6,5 | 100,0 | 3:1 |
| 19 | aus Beispiel 11 | 3,3 | 99,5 | 3:1 |
| 20 | aus Beispiel 5 | 24 | 89,3 | 1:1 |

*) Typ XZO 631/02, MEL Chemicals, Manchester, UK ($ZrO_2 \cdot x\ H_2O$)

**Beispiele 21—23, Vergleichsbeispiel 4**

**1-(*p*-Chlorphenyl)ethanol (Meerwein—Ponndorf—Verley-Reduktion)**

In einem Rundkolben mit Magnetrührer und Rückflusskühler bzw. einem 100 ml Autoklav wurden 5,0 g (32,3 mmol) *p*-Chloracetophenon, 45 g Isopropylalkohol und 1,0 g Katalysator zum Rückfluss bzw. auf 120 °C erhitzt. Nach dem Ende der Reaktionsdauer wurde das Reaktionsgemisch gaschromatographisch analysiert und die Ausbeute bestimmt. Die Ergebnisse sind in der folgenden Tabelle 3 zusammengefasst.

Tabelle 3

| Beispiel Nr. | Katalysator | Reaktionsdauer*) [h] | Ausbeute [%] |
|---|---|---|---|
| V 4 | aus Beispiel 1 (undotiert) | 24 (R) | 22,5 |
| 21 | aus Beispiel 4 | 24 (R) | 38,9 |
| 22 | aus Beispiel 9 | 24 (R) | 69,7 |
| 23 | aus Beispiel 9 | 16 (A) | 94,2 |

*) (R) = Rückfluss; (A) = Autoklav

Der Versuch von Beispiel 22 wurde ausserdem noch fünfmal mit derselben Katalysatorprobe wiederholt, wobei nach jeder Wiederholung der Kupfergehalt des Katalysators kontrolliert wurde. Es wurde weder eine signifikante Ausbeuteveränderung noch eine Verminderung des Kupfergehalts im Katalysator beobachtet.

**Beispiele 24—25, Vergleichsbeispiel 5**

**3-Methyl-2-buten-1-ol (Meerwein—Ponndorf—Verley-Reduktion)**

In einem Rundkolben mit Magnetrührer und Rückflusskühler bzw. einem 100 ml Autoklav wurden 3,0 g (35,7 mmol) 3-Methyl-2-butenal, 72 g Isopropylalkohol und 1,0 g Katalysator zum Rückfluss bzw. auf 110 °C erhitzt. Nach dem Ende der Reaktionsdauer wurde das Reaktionsgemisch gaschromatographisch analysiert und die Ausbeute bestimmt. Die Ergebnisse sind in der folgenden Tabelle 4 zusammengefasst.

Tabelle 4

| Beispiel Nr. | Katalysator | Reaktionsdauer*) [h] | Ausbeute [%] |
|---|---|---|---|
| V 5 | aus Beispiel 2 (undotiert) | 24 (R) | 3,8 |
| 24 | aus Beispiel 10 | 30 (R) | 86,5 |
| 25 | aus Beispiel 10 | 18 (A) | 91,5 |

*) (R) = Rückfluss; (A) = Autoklav

**Beispiele 26—27, Vergleichsbeispiel 6**

**Zimtaldehyd (Oppenauer-Oxidation)**

Unter Argon wurden 1,5 g (11,2 mmol) Zimtalkohol (*trans*-3-Phenyl-2-propen-1-ol), 36 g (367 mmol) Cyclohexanon, 0,5 g Katalysator und 1,5 g Dodecan (als GC-Standard) erhitzt. Nach dem Ende der Reaktionsdauer wurde das Reaktionsgemisch gaschromatographisch analysiert und die Ausbeute bestimmt. Die Ergebnisse sind in der folgenden Tabelle 5 zusammengefasst.

Tabelle 5

| Beispiel Nr. | Katalysator | Reaktionsdauer [h] | Temperatur [°C] | Ausbeute [%] |
|---|---|---|---|---|
| V 6 | MEL*) (undotiert) | 24 | 100 | 37,1 |
| 26 | aus Beispiel 10 | 24 | 60 | 56,4 |
| 27 | aus Beispiel 9 | 5,5 | 120 | 67,2 |

*) Typ XZO 631/02, MEL Chemicals, Manchester, UK ($ZrO_2 \cdot x\ H_2O$)

**Beispiel 28**

**Acetophenon (Oppenauer-Oxidation)**

Unter Argon wurden 5,0 g (40,9 mmol) 1-Phenylethanol, 15,0 g (153 mmol) Cyclohexanon und 1,0 g Katalysator aus Beispiel 9 in 30 g Toluol auf 70 °C erhitzt. Nach 10 h Reaktionsdauer wurde das Reaktionsgemisch gaschromatographisch analysiert. Ausbeute: 94,2%.

**Beispiel 29, Vergleichsbeispiel 7**

**Benzaldehyd (Oppenauer-Oxidation)**

Unter Argon wurden 1,5 g (13,9 mmol) Benzylalkohol, 3,0 g (27,8 mmol) *p*-Benzochinon und 0,5 g Katalysator in 26 g 1,4-Dioxan zum Rückfluss erhitzt. Nach 24 h Reaktionsdauer wurde gaschromatographisch die Ausbeute bestimmt. Mit dem erfindungsgemässen Katalysator aus Beispiel 10 betrug die Ausbeute 55,6%; mit dem nicht erfindungsgemässen undotierten Katalysator aus Beispiel 2 wurden nur 23,6% Ausbeute erhalten.

**Beispiel 30**

**3-Hydroxychinuclidin**

Ein 100 ml Autoklav („Magnedrive II", Autoclave Engineers Europe) mit Hohlschaft-Intensivrührer (Dispersimax®) wurde mit 5,0 g Chinuclidin-3-on (hergestellt aus dem Hydrochlorid durch Umsetzung mit Natriummethylat und Extraktion mit Diethylether, 45 ml Isopropylalkohol und 0,25 g Katalysator (aus Beispiel 1) beschickt, mit Stickstoff gespült und bei Raumtemperatur unter 10 bar Stickstoffdruck gesetzt. Unter Rühren (1500 $min^{-1}$) wurde auf 200 °C geheizt und 8 h bei dieser Temperatur belassen. Die Ausbeute wurde mittels GC zu 97,3% bestimmt. Eine Wiederholung des Ansatzes mit demselben Katalysator lieferte nach 13 h Reaktionszeit noch 92,8% Ausbeute.

**Beispiel 31**

**3-Hydroxychinuclidin**

In einem Autoklaven wurden 81,6 g (0,496 mol) Chinuclidin-3-on-Hydrochlorid und 12,4 g Katalysator (aus Beispiel 1) unter kräftigem Rühren in 800 ml Isopropylalkohol suspendiert. Der Autoklav wurde verschlossen und die enthaltene Luft unter Normaldruck durch Argon verdrängt. Dann wurde der Autoklav auf 150 °C Innentemperatur erhitzt, wobei sich ein Druck von 6—8 bar einstellte, und 7 h auf dieser Temperatur gehalten. Nach dem Abkühlen auf Raumtemperatur wurden 89,5 g einer 30%igen Lösung von Natriummethylat in Methanol zugegeben. Das Gemisch wurde noch 45 min gerührt und dann filtriert. Das Filtrat wurde im Vakuum eingeengt und der Rückstand aus 640 ml Toluol heiss umkristallisiert. Der Katalysator wurde mit Wasser salzfrei gewaschen und getrocknet, wonach er wiederverwendet werden konnte.

Ausbeute: 59,2 g (93%), Gehalt (GC) 99%

**Patentansprüche**

1. Katalysatorzusammensetzung auf Basis von amorphem teildehydratisiertem Zirconiumhydroxid, dadurch gekennzeichnet, dass das amorphe teildehydratisierte Zirconiumhydroxid mit 0,01 bis 20 Atom-% Kupfer und/oder 0,01 bis 20 Atom-% Nickel, jeweils bezogen auf Zirconium, dotiert ist und eine spezifische Oberfläche nach BET von mindestens 50 $m^2$/g besitzt.

2. Katalysatorzusammensetzung nach Anspruch 1, gekennzeichnet durch die Formel

$$Cu_aNi_bZrO_{2+a+b} \cdot x\, H_2O,$$

worin $0 \le a \le 0{,}2$; $0 \le b \le 0{,}2$ und $0{,}2 \le x \le 2{,}0$ ist, mit der Massgabe, dass $a + b \ge 0{,}0001$ ist.

3. Katalysatorzusammensetzung nach Anspruch 2, dadurch gekennzeichnet, dass $a \le 0{,}15$; $b \le 0{,}1$; $a + b \ge 0{,}000\,2$ und $0{,}3 \le x \le 2{,}0$ ist.

4. Katalysatorzusammensetzung nach einem der Ansprüche 1 bis 3, dadurch gekennzeichnet, dass die spezifische Oberfläche nach BET grösser als 100 $m^2/g$ ist.

5. Verfahren zu Herstellung einer Katalysatorzusammensetzung gemäss einem der Ansprüche 1 bis 4, dadurch gekennzeichnet, dass aus einer wässrigen Lösung eines Zirconiumsalzes durch Zugabe einer Base Zirconiumhydroxid ausgefällt, bei 200 bis 400 °C kalziniert und anschliessend mit einer Lösung eines Kupfer- und/oder Nickelsalzes imprägniert und getrocknet wird.

6. Verfahren nach Anspruch 5, dadurch gekennzeichnet, dass als Zirconiumsalz Zirconylchlorid und als Base wässrige Ammoniaklösung oder eine wässrige Alkalihydroxidlösung eingesetzt wird.

7. Verfahren nach Anspruch 5 oder 6, dadurch gekennzeichnet, dass als Kupfer- und/oder Nickelsalz das entsprechende Nitrat eingesetzt wird.

8. Verfahren nach einem der Ansprüche 5 bis 7, dadurch gekennzeichnet, dass die Kalzination bei 250 bis 350 °C durchgeführt wird.

9. Verfahren zur Reduktion von Aldehyden oder Ketonen zu den entsprechenden primären bzw. sekundären Alkoholen durch Wasserstoffübertragung von einem sekundären Alkohol als Wasserstoffdonor in Gegenwart eines Katalysators, dadurch gekennzeichnet, dass als Katalysator eine Katalysatorzusammensetzung gemäss einem der Ansprüche 1 bis 4 eingesetzt wird.

10. Verfahren nach Anspruch 9, dadurch gekennzeichnet, dass als Wasserstoffdonor Isopropylalkohol eingesetzt wird.

11. Verfahren zur Oxidation von primären oder sekundären Alkoholen zu den entsprechenden Aldehyden bzw. Ketonen durch Wasserstoffübertragung auf ein Keton oder Chinon als Wasserstoffakzeptor in Gegenwart eines Katalysators, dadurch gekennzeichnet, dass als Katalysator eine Katalysatorzusammensetzung gemäss einem der Ansprüche 1 bis 4 eingesetzt wird.

12. Verfahren nach Anspruch 11, dadurch gekennzeichnet, dass als Wasserstoffakzeptor Cyclohexanon oder *p*-Benzochinon eingesetzt wird.

13. Verfahren zur Herstellung von 3-Hydroxychinuclidin der Formel

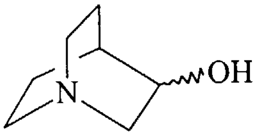

I

oder eines entsprechenden Salzes durch Reduktion von Chinuclidin-3-on der Formel

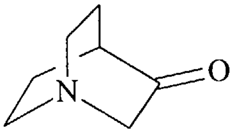

II,

oder eines entsprechenden Salzes, dadurch gekennzeichnet, dass dieses mit einem sekundären Alkohol als Wasserstoffdonor in Gegenwart von amorphem teildehydratisiertem Zirconiumhydroxid umgesetzt wird.

14. Verfahren nach Anspruch 13, dadurch gekennzeichnet, dass als sekundärer Alkohol Isopropylalkohol eingesetzt wird.

15. Verfahren nach Anspruch 13 oder 14, dadurch gekennzeichnet, dass die Reaktion bei 120—220 °C, vorzugsweise bei 150—200 °C, in der Flüssigphase durchgeführt wird.

Europäisches Patentamt

# EUROPÄISCHER RECHERCHENBERICHT

Nummer der Anmeldung
EP 98 10 3948

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | US 4 511 455 A (DOSCH ROBERT G ET AL)<br>* Spalte 3, Zeile 45-46 *<br>* Spalte 4, Zeile 45-46 *<br>* Beispiel 6 *<br>--- | 1-8 | B01J21/06<br>B01J23/72<br>B01J23/755<br>C07C45/29<br>C07C29/14<br>C07C29/143 |
| P,X | WO 98 00223 A (LOW EMISSIONS TECHNOLOGIES RES)<br>* Ansprüche 1-3; Beispiele 1-3 *<br>--- | 1-8 | |
| A | EP 0 603 409 A (JAPAN TOBACCO INC)<br>--- | 1-15 | |
| A | EP 0 596 108 A (JAPAN TOBACCO INC)<br>--- | 1-15 | |
| A | US 4 877 909 A (MIZUSAKI SIGENOBU ET AL)<br>----- | 1-15 | |

RECHERCHIERTE SACHGEBIETE (Int.Cl.6)
B01J
C07C

Der vorliegende Recherchenbericht wurde für alle Patentansprüche erstellt

| Recherchenort | Abschlußdatum der Recherche | Prüfer |
|---|---|---|
| MÜNCHEN | 6.Mai 1998 | Schwaller, J-M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)